(19) Europäisches Patentamt

European Patent Office

Office européen des brevets

(11) EP 0 871 738 B1

(12) FASCICULE DE BREVET EUROPEEN

(45) Date de publication et mention
de la délivrance du brevet:
**15.02.2006 Bulletin 2006/07**

(51) Int Cl.:
*C12N 15/33* (2006.01)     *C12N 15/35* (2006.01)
*C12N 15/40* (2006.01)     *C07K 14/015* (2006.01)
*A61K 39/295* (2006.01)     *C12N 15/36* (2006.01)

(21) Numéro de dépôt: **96930192.8**

(22) Date de dépôt: **30.08.1996**

(86) Numéro de dépôt international:
**PCT/FR1996/001337**

(87) Numéro de publication internationale:
**WO 1997/024443 (10.07.1997 Gazette 1997/30)**

(54) **PSEUDO-PARTICULES VIRALES RECOMBINANTES ET APPLICATIONS VACCINALES ET ANTITUMORALES**

REKOMBINANTE VIRALE PSEUDOPARTIKEL UND DEREN VERWENDUNG ALS IMPFSTOFF ODER ALS ANTITUMORALE WIRKSTOFFE

VIRAL RECOMBINANT PSEUDO-PARTICLES AND VACCINAL AND ANTI-TUMORAL APPLICATIONS

(84) Etats contractants désignés:
**AT BE CH DE DK ES FI FR GB GR IE IT LI LU MC NL PT SE**

(30) Priorité: **02.01.1996 EP 96400009**

(43) Date de publication de la demande:
**21.10.1998 Bulletin 1998/43**

(73) Titulaires:
• **INSTITUT PASTEUR**
  **75724 Paris Cédex 15 (FR)**
• **Immunologia Y Genetica Aplicada, S.A.**
  **28037 Madrid (ES)**

(72) Inventeurs:
• **CASAL, Ignacio**
  **E-22039 Madrid (ES)**
• **SEDLIK, Christine**
  **F-95100 Argenteuil (FR)**
• **SARRASECA, Javier**
  **E-28041 Madrid (ES)**
• **LECLERC, Claude**
  **F-75015 Paris (FR)**
• **LO MAN, Richard**
  **F-75015 Paris (FR)**
• **RUEDA, Paloma**
  **E-28011 Madrid (ES)**

(74) Mandataire: **Phélip, Bruno et al**
  **c/o Cabinet Harlé & Phélip**
  **7, rue de Madrid**
  **75008 Paris (FR)**

(56) Documents cités:
  **WO-A-91/04330**

• **JOURNAL OF GENERAL VIROLOGY, vol. 76, no. 9, Septembre 1995, READING GB, pages 2361-2368, XP002005309 C.SEDLIK ET AL.: "Immunogenicity of poliovirus B and T cell epitopes presented by hybrid porcine parvovirus particles" cité dans la demande**
• **PROCEEDINGS OF THE NATIONAL ACADEMY OF SCIENCES OF USA, vol. 91, Août 1994, WASHINGTON US, pages 8507-8511, XP002005310 K.MIYAMURA ET AL.: "Parvovirus particles as platforms for protein presentation"**
• **JOURNAL OF VIROLOGY, vol. 69, no. 4, Avril 1995, pages 2187-2193, XP002005311 D.MOSKOPHIDIS ET R.M.ZINKERNAGEL: "Immunobiology of cytotoxic T- cell escape mutants of lymphocytic choriomeningitis virus"**

Remarques:
Le dossier contient des informations techniques présentées postérieurement au dépôt de la demande et ne figurant pas dans le présent fascicule.

EP 0 871 738 B1

**Description**

La présente invention concerne des pseudo-particules virales recombinantes d'un virus de la famille des *Parvoviridae* ou d'un virus apparenté, utiles notamment pour induire des réponses lymphocytaires TCD8+ cytotoxiques (CTL) *in vivo* à un niveau élevé.

Elle a encore pour objet l'utilisation de ces pseudo-particules pour la fabrication de vaccins ou de médicaments anti-tumoraux.

Elle a également trait à des compositions comprenant lesdites pseudo-particules dans un véhicule et / ou un diluant physiologiquement acceptable.

Selon Fernandez et al. (Médecine/Sciences, 1995, 11, 975-983), la réponse à médiation cellulaire, essentielle dans le cas d'une tumeur, peut être divisée en deux parties:

- d'une part la mise en route de la réponse qui fait intervenir des lymphocytes TCD4+ et des cellules présentatrices de l'antigène (CPA), et
- d'autre part la réponse effectrice cytotoxique dans laquelle interviennent les lymphocytes TCD8+.

Les lymphocytes TCD4+ activés vont proliférer et secréter des cytokines alors que les lymphocytes TCD8+ activés vont proliférer et se différencier en lymphocytes T cytotoxiques (CTL).

Ces deux familles de lymphocytes ont donc des fonctions très différentes et sont activées par des mécanismes distincts. La stimulation des lymphocytes CD4+ nécessite l'association d'un peptide issu de la dégradation de l'antigène à des molécules de classe II du Complexe Majeur d'Histocompatibilité (CMH), alors que celle des cellules CD8+ nécessite l'association d'un peptide à des molécules de classe I du CMH.

On a déjà proposé un certain nombre de stratégies prophylactiques ou thérapeutiques pour lutter contre des maladies d'origines diverses chez l'homme et l'animal causées par des virus, des bactéries, des parasites ou contre des maladies cancéreuses ou tumorales.

On connaît, par exemple, l'induction de réponses CTL *in vivo* par des vecteurs " vivants ". Cependant l'innocuité de ces vecteurs n'est pas garantie, notamment chez des individus immunodéprimés. D'autre part, les systèmes impliquant la réplication de ces vecteurs " vivants " ne conservent pas leur efficacité chez des individus immuns.

Martinez et al., enseignent dans Vaccine (vol. 10, pp. 684-690, 1992) l'utilisation de capsides vides de parvovirus de porc (PPV) dans la vaccination des porcs. La famille des *Parvoviridae* regroupe des virus distribués très largement chez des mammifères tels que porcs, bovins, chats, lapins, rats et chez l'homme. Cependant, les parvovirus sont relativement spécifiques des mammifères hôtes. Le parvovirus de porc (PPV), en particulier, est responsable de nombreuses infections dans les élevages industriels de porcs. Le parvovirus de porc (PPV) est constitué d'une particule isométrique non enveloppée de 20 nm de diamètre et à symétrie icosaédrique, contenant une molécule d'ADN simple brin. Il est composé de deux protéines de capsides, VP1 (83 kDa) et VP2 (64 kDa), et d'une troisième protéine, VP3 résultant de la protéolyse de VP2.

La protéine VP2 peut être produite par des cellules d'insectes à l'aide d'un système de baculovirus recombinant et les protéines VP2 obtenues sont capables de s'auto-assembler pour former des pseudo-particules virales qui ont la même taille que celle du virion natif. La vaccination de porcs contre le PPV est réalisée en les immunisant avec un mélange desdites capsides avec l'association adjuvante Alhydrogel (à 50 %) + Quil A 500 μg (Superfos). Un enseignement comparable à celui de cet article est retrouvé dans les demandes EP-551.449 et EP-554.414 qui indiquent de plus que des épitopes correspondant à d'autres protéines virales peuvent être incorporés aux capsides, sans toutefois préciser la nature de ces épitopes.

La demande EP-647.655 est quant à elle relative à des peptides synthétiques correspondant à des sites antigéniques de VP2. Il ne s'agit donc pas de la protéine entière.

Sedlik et al. décrivent, dans Journal of General Virology (76 : 2361-2368, (1995)), l'utilisation de particules hybrides de PPV comme vecteurs de deux épitopes du virus de la polio, C3 : B et C3 : T reconnus respectivement par des lymphocytes B et TCD4+. Les particules contenant l'épitope C3 : TCD4+ ne se révèlent capables de stimuler les réponses prolifératives que de cellules T, CD4+ et non de cellules T CD8+. Outre cette spécificité de stimulation, les réponses restent d'un niveau peu élevé, insuffisant pour une immunisation efficace. En tout état de cause, l'induction de ces réponses requiert obligatoirement l'utilisation d'un adjuvant (hydroxyde d'aluminium ou adjuvant complet de Freund).

En outre, les essais de modification des pseudo-particules en vue d'incorporer des épitopes hétérologues se heurtent à des difficultés comme l'inhibition de la formation des pseudo-particules . Même lorsque ces dernières se forment, l'épitope hétérologue n'est pas nécessairement immunogène, l'épitope pouvant par exemple se situer dans un site où il est incapable de prendre sa conformation naturelle ou d'être produit par les cellules présentant l'antigène.

Par ailleurs à l'instar des vaccins à vecteurs " vivants ", l'innocuité de ces particules hybrides associées aux adjuvants n'est pas totalement assurée, une toxicité pouvant provenir de certaines propriétés des adjuvants susceptibles d'induire des effets secondaires néfastes pour l'organisme.

Il ressort donc de l'état de la technique que l'on ne connaissait pas de système fiable, efficace permettant de stimuler la réponse lymphocytaire T-cytotoxique et T auxiliaire, sans risque pour la santé de l'individu traité et sans utilisation d'adjuvants immunostimulateurs.

En outre, des particules hybrides contenant l'épitope C3: T ne se révélaient pas capables d'induire une réponse T CD4+ efficace, en l'absence d'adjuvant.

Le demandeur a montré qu'il est possible d'induire spécifiquement sans utiliser d'immunoadjuvants une réponse lymphocytaire CD8+, c'est-à-dire une réponse cytotoxique, à l'aide de pseudo-particules portant des épitopes susceptibles de s'associer avec au moins une molécule de classe I du CMH, respectivement.

La présente invention a pour objet des pseudo-particules virales recombinantes caractérisées en ce qu'elles sont constituées par auto-assemblage d'au moins la protéine de structure virale VP2 d'un virus de la famille des *Parvoviridae*, ou d'un virus apparenté, en ce qu'elles ont une taille comprise entre environ 20 et 60 nm, en ce qu'elles forment une structure approximativement icosaédrique, et en ce que la protéine VP2 est modifiée, en son extrémité N-terminale, par la présence d'au moins un épitope comprenant une séquence de 8 à 25 acides aminés susceptible de s'associer à au moins une molécule de classe I du complexe majeur d'histocompatibilité, ladite association étant reconnue par des lymphocytes T cytotoxiques.

Les lymphocytes TCD8+ selon l'invention sont capables de reconnaître au moins un épitope comprenant une séquence de 8 à 9 acides aminés susceptible de s'associer à au moins une molécule de classe I du CMH.

Les lymphocytes TCD4+ selon l'invention sont capables de reconnaître une séquence comprise entre 8 et 25 acides aminés susceptible de s'associer à au moins une molécule de classe II du CMH.

Les particules selon l'invention peuvent comprendre des protéines de diverses natures, et former des particules hybrides. Elles peuvent par exemple être constituées de protéines VP2 et VP1.

L'association formée entre la séquence correspondant à l'épitope et les molécules de classe I est reconnue par le récepteur de lymphocytes T CD8+ et induit une différenciation et une prolifération de ces lymphocytes.

Avantageusement, ladite séquence est bordée de régions dites flanquantes susceptibles de moduler sa dégradation, c'est-à-dire de faciliter sa production lors de la dégradation de l'antigène, ou de faciliter sa fixation aux molécules de classe I du CMH.

Les régions flanquantes peuvent correspondre aux acides aminés flanquant l'épitope T, CD8+ dans son environnement naturel. De manière générale, elle peuvent être composées de plusieurs acides aminés, et en particulier d'alanine ou de lysine.

Les pseudo-particules conformes à l'invention présentent un premier intérêt, provenant de la sécurité de ce mode d'immunisation, le vecteur mis en oeuvre étant non-réplicatif, constitué d'un seul type de protéine virale et pouvant être administré même à des individus immunodéprimés.

Un second avantage des pseudo-particules selon l'invention réside dans l'absence de réactions croisées avec le parvovirus humain et donc dans l'absence de problèmes liés à une pré-immunisation des individus, aboutissant éventuellement à une faible immunogénicité de ce type de vaccins recombinants.

En outre les pseudo-particules selon l'invention ne nécessitent pas la réplication du vecteur et peuvent donc conserver leur efficacité chez des individus immuns.

Egalement, les pseudo-particules conformes à l'invention possèdent une excellente immunogénicité - une seule immunisation suffit - et peuvent donc être utilisées en l'absence d'adjuvant immunostimulateur.

Avantageusement, le parvovirus est le PPV, le CPV (Canine Parvovirus) ou un autre virus apparenté tel que le FPLV (Feline Panleukopenia virus) et le MEV (Mink Enteritis virus).

L'épitope peut être constitué par toute séquence se fixant à une molécule du CMH de classe I et susceptible d'induire une cytotoxicité T lymphocytaire. Il peut être en particulier un épitope TCD8+ d'un virus, tel que le VIH-1 ou le VIH-2, ou le virus de la grippe, ou un épitope tumoral ou exprimé par des cellules cancéreuses.

Selon un mode avantageux de réalisation, les pseudo-particules selon l'invention sont caractérisées en ce que l'épitope sélectionné est l'épitope CTL CD8+ contenu dans la région 118-132 de la nucléoprotéine du virus de la choriomeningite lymphocytaire (LCMV).

L'épitope peut néanmoins être tout autre épitope susceptible d'induire une réponse CTL, tel que par exemple l'un des épitopes suivants:

- Les épitopes des protéines Env gp120, Env gp41, Gag p17, Gag p24, Gag p15, Pol et Nef, des virus VIH et les épitopes des protéines Gag, et nef du virus VIS et Gag du virus VIH-2, tels que définis par VENET and WALKER (AIDS 1993, Vol.7, suppl.1, 119-120).
- les épitopes de protéines exprimées par des tumeurs se développant chez l'être humain, telles que les protéines exprimées par les gènes MAGE-1, MAGE-3, BAGE, GAGE-1,2, HER-2/neu, et d'antigènes de différenciation mélanocytique, tels que la tyrosinase, Pmel17gp100, Melan-AMART-1 , et gp 75TRP1, définis par VAN DEN EYNDE et BRICHARD (Current Opinion in Immunology, 1995, 7, 674-681).

Afin d'induire aussi une réponse T CD4$^+$ auxiliaire ou résultant dans la production de lymphokines, l'épitope peut être l'épitope T CD4$^+$ PréS:T situé dans la région PréS2 (séquence 120-132) du virus de l'hépatite B (HBV).

La pseudo-particule peut aussi comprendre une combinaison d'épitopes, et en particulier:

- au moins un épitope TCD4$^+$ et au moins un épitope TCD8$^+$
- une combinaison de multicopies d'épitopes TCD4$^+$ ou TCD8$^+$,
- une combinaison de quatre copies d'épitopes TCD8$^+$ Mut du carcinome de Lewis.

Par combinaison d'épitopes, on entend une combinaison comprenant au moins deux épitopes et de façon préférée entre deux et dix épitopes de protéines identiques comme par exemple une multicopie d'épitopes Mut1 du carcinome de Lewis ou de protéines différentes. Ainsi, une combinaison multicopies d'épitopes TCD8$^+$ peut comprendre au moins deux épitopes et de façon préférée entre deux et dix épitopes issus de protéines différentes comme par exemple l'association d'un épitope TCD8$^+$ de la nucléoprotéine de la chorioméningite lymphocytaire et d'un épitope TCD8$^+$ Env. gp 41 du VIH.

De manière avantageuse, lesdites pseudo-particules virales recombinantes sont susceptibles d'être obtenues par un procédé comprenant une étape d'expression dans le baculovirus, de la protéine chimérique constituée de la protéine VP2 du parvovirus et dudit épitope.

Les pseudo-particules conformes à l'invention sont obtenues, de façon encore plus préférée, par auto-assemblage de la protéine VP2 de parvovirus modifiée par la présence, à son extrémité N-terminale, d'au moins un épitope de la nucléoprotéine de LCMV ou d'au moins un épitope de l'HBV ou encore d'une combinaison de multicopies d'épitopes.

On notera que toute autre molécule, et en particulier toute autre protéine, présentant des propriétés similaires à VP2, telle que l'auto-assemblage, et pouvant être modifiées par un épitope, peuvent être utilisées dans le cadre de la présente invention en remplacement de VP2.

La présente invention a aussi pour objet l'utilisation des pseudo-particules en quantité immunisante efficace, pour l'induction de réponses CTL *in vivo* à un niveau élevé.

Par l'expression " quantité immunisante efficace ", on entend une quantité choisie en fonction de la voie d'administration et du poids de l'individu de pseudo-particules selon l'invention. Avantageusement la quantité administrée sera comprise entre 10 et 500$\mu$g de pseudo-particules par individu.

La présente invention a en outre pour objet une composition comprenant les pseudo-particules dans un véhicule et/ou un diluant acceptables d'un point de vue immunologique. Comme diluant on peut utiliser une solution aqueuse tamponnée au voisinage de pH 7 (soluté physiologique).

De manière avantageuse, la composition conforme à la présente invention est exempte d'adjuvant immunostimulant. Elle peut néanmoins contenir, si nécessaire, un tel adjuvant qui peut être de l'hydroxyde d'aluminium.

Les pseudo-particules selon la présente invention peuvent aussi être utilisées dans un procédé de stimulation in vitro des réponses T cytotoxiques de lymphocytes provenant de sujets atteints d'infections virales ou tumorales comprenant la mise en contact des lymphocytes des sujets avec les pseudo-particules. Dans ce cas, les cellules sont, après traitement, réadministrées aux patients, par exemple par injection.

La présente invention a encore pour objet l'utilisation des pseudo-particules conformes à l'invention dans la préparation d'un vaccin, de préférence à dose unique, notamment antitumoral ou antiviral. Il peut être administré par les voies habituelles pour les vaccins, à savoir par voie intramusculaire, intradermique, sous-cutanée ou éventuellement par voie orale ou toute autre voie permettant l'activation d'une réponse CTL.

L'invention est illustrée sans être aucunement limitée par la description qui suit, faite en référence aux dessins annexés sur lesquels:

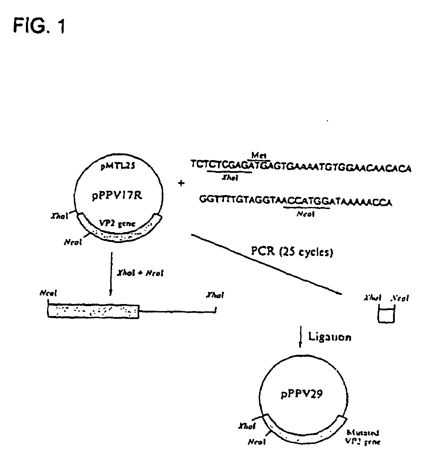

La figure 1 représente le procédé d'obtention du plasmide pPPV29.

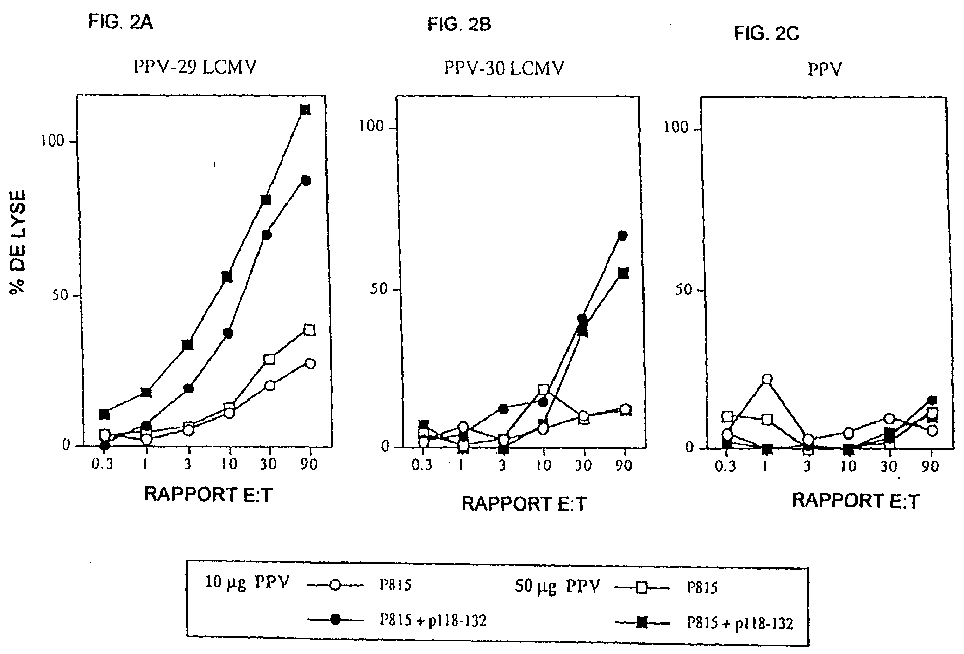

La Figure 2 comprenant trois graphiques 2 (a), 2(b) et 2(c) illustre l'immunisation de souris, à deux reprises, à j0 et j21 avec respectivement 10 et 50 $\mu$g de pseudo-particules conformes à l'invention, sans adjuvant A j28, les splénocytes sont stimulés *in vitro* pendant 5 jours avec des splénocytes syngéniques pré-incubés avec le peptide synthétique 118-132. L'activité lymphocytaire T cytotoxique est mesurée sur des cibles P815 pré-incubées avec du milieu ou avec le peptide synthétique 118-132. Les résultats sont exprimés en % de lyse, le rapport E:T étant porté en abscisse, cette expression E/T désignant le rapport lymphocytes effecteurs/cellules cibles. La figure 2(a) concerne les résultats obtenus avec la particule PPV-29LCMV, la figure 2(b) les résultats obtenus avec la particule PPV-30LCMV et la figure 2(c) à titre comparatif avec les pseudo-particules non modifiées.

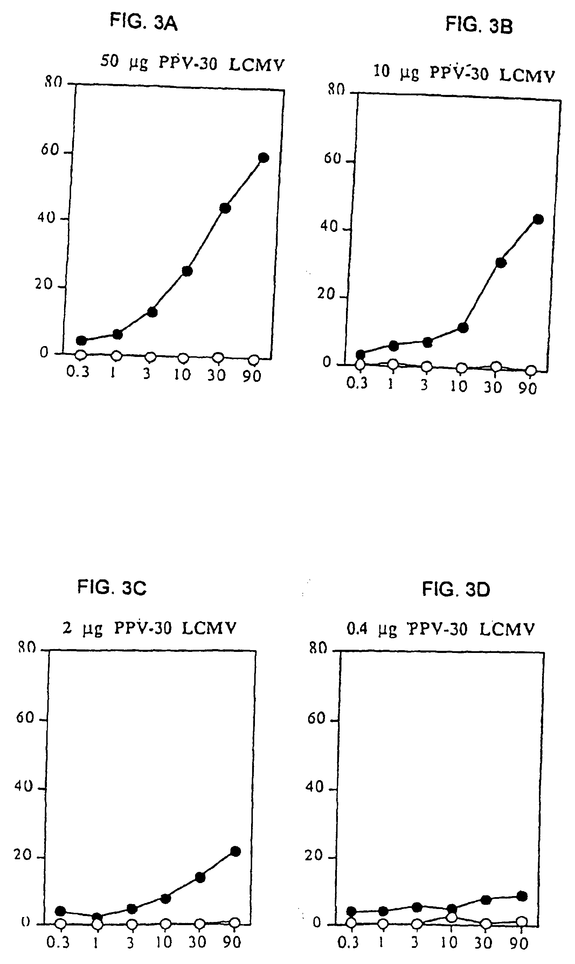

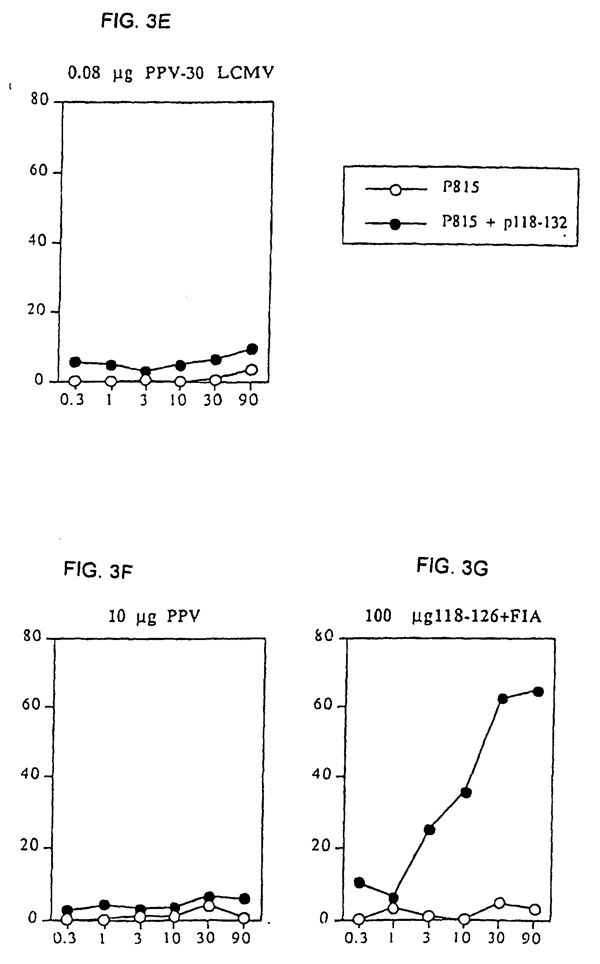

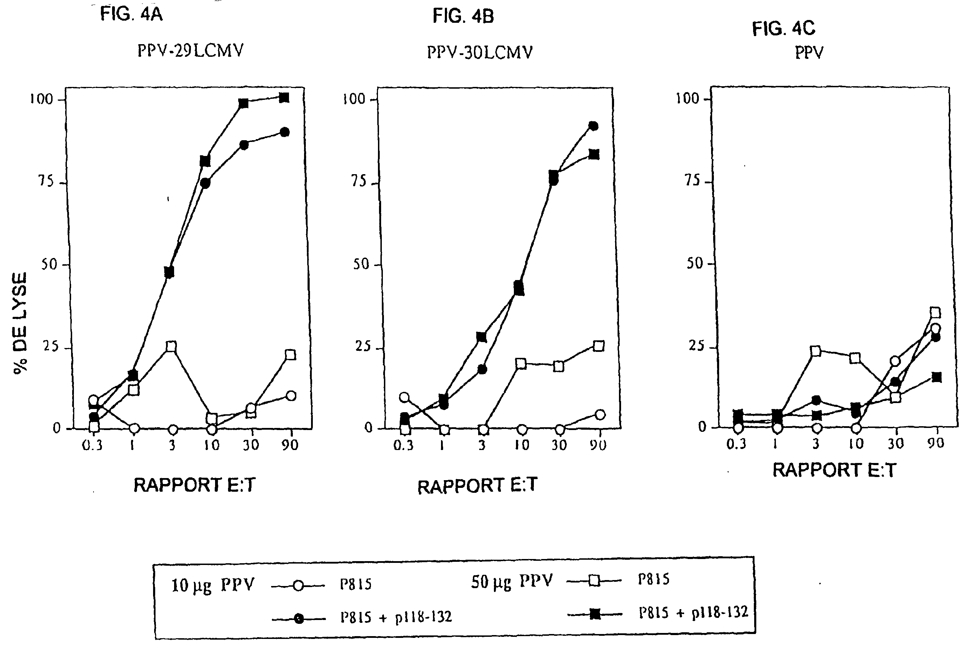

La figure 3 comprenant 7 graphiques 3(a), 3(b), 32(c), 3(d), 3(e), 3(f) et 3 (g) illustre l'immunisation de souris, à deux reprises, à j0 et j21, avec différentes doses respectivement de 50 $\mu$g pour la figure 3(a), 10 $\mu$g pour la figure 3(b), 2 $\mu$g pour la figure 3(c), 0,4 $\mu$g pour la figure 3(d), 0,08 $\mu$g pour la figure 3(e) de pseudo-particules selon l'invention, sans adjuvant. A j28, les splénocytes sont stimulés *in vitro* pendant 5 jours avec des splénocytes syngéniques pré-incubés avec le peptide synthétique 118-132. L'activité lymphocytaire T cytotoxique est mesurée sur des cibles P815 pré-incubées avec du milieu ou avec le peptide synthétique 118-132. Les souris témoins ont reçu une injection à j21 de 100 $\mu$g de peptide synthétique 118-126 en adjuvant incomplet de Freund (IFA) (fig. 3g) ou de pseudo-particules témoins (fig. 3f). Les résultats sont exprimés en % de lyse, de la même manière qu'à la figure 2. La figure 4 comprenant, d'une manière similaire à la Figure 2, trois graphiques 4(a), 4(b) et 4(c) illustre l'immunisation de souris par une seule injection de 10 $\mu$g ou de 50 $\mu$g de pseudo-particules conformes à l'invention, sans adjuvant. A j14, les splénocytes sont stimulés *in vitro* pendant 5 jours avec des spténocytes syngéniques pré-incubés avec le peptide synthétique 118-132. L'activité lymphocytaire T cytotoxique est mesurée sur des cibles P815 pré-incubées avec du milieu ou avec le peptide synthétique 118-132. Les résultats sont exprimés en % de lyse, de la même manière qu'à la Figure 2.

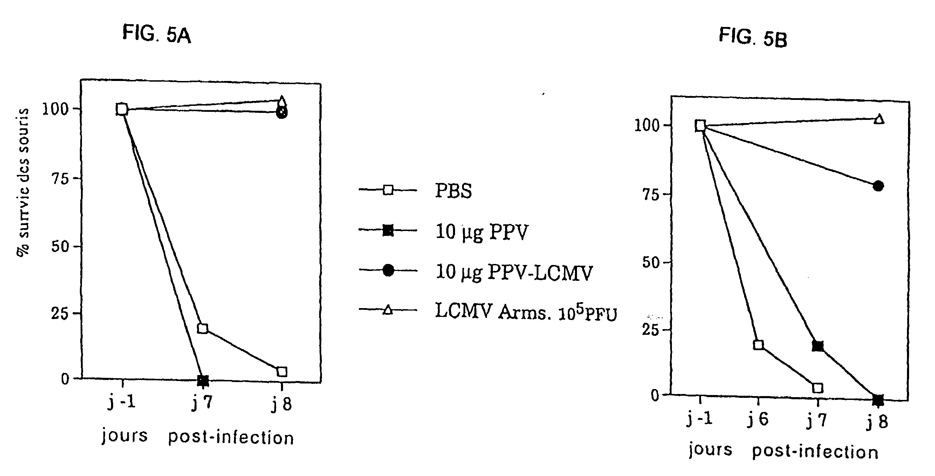

La figure 5 comprenant deux graphiques 5(a) et 5(b) illustre la protection de souris BALB/c, injectées par voie intrapéritonéale (i.p.) aux jours 0 et 21 avec du PBS, ou avec des pseudo-particules vides (PPV) ou exprimant la séquence 118-132 de la nucléoprotéine du LCMV (PPV-LCMV), ou avec le virus souche Armstrong (LCMV-Arms), contre l'infection par LCMV respectivement aux jours 28 (5a) et 70 (5b). Les souris ont été infectées par voie intracérébrale avec $10^{1,7}$PFU/souris. La mortalité des souris est suivie quotidiennement pendant 10 jours et les résultats sont exprimés en % de survie parmi les animaux de chaque lot.

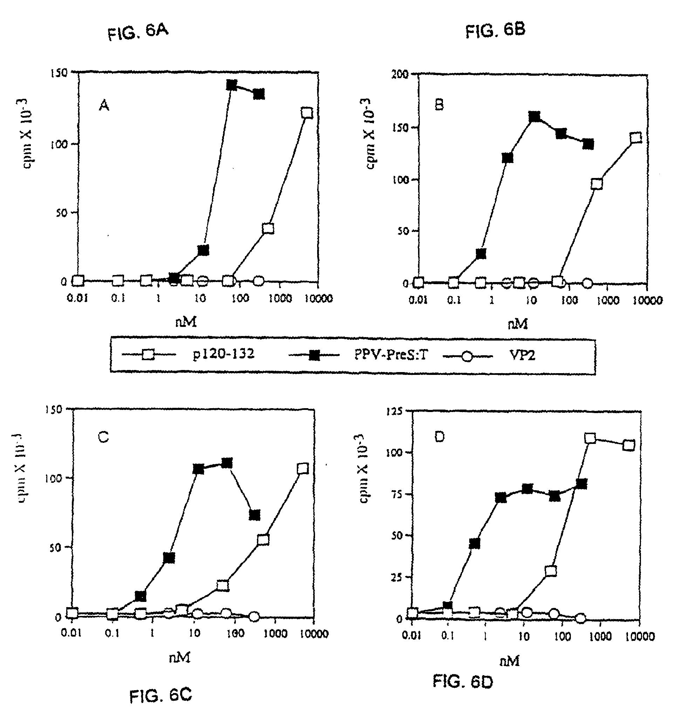

Les figures 6A à 6D illustrent la stimulation d'hybridomes T spécifiques de l'épitope T CD4[+] PréS:T de HBV, 51 E12 (6A et 6C) ou 52A12 (6B et 6D) par des cellules de lymphome B (6A et 6B) ou des splénocytes irradiés (6C et 6D) mises en présence de peptide PréS:T, de pseudo-particules recombinantes PPV-PréS:T ou de pseudo-particules PPV (VP2).

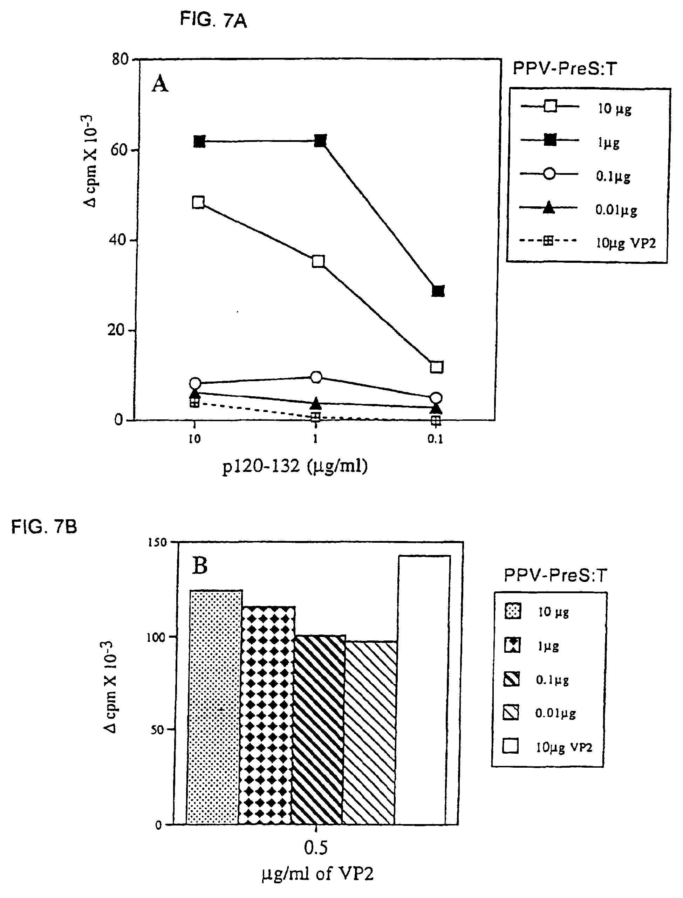

Les figures 7A et 7B illustrent la réponse proliférative de cellules ganglionnaires stimulées in vitro avec du peptide PréS:T (figure 7A) ou des pseudo-particules PPV (VP2) (figure 7B), lesdites cellules étant issues de souris DBA/1 immunisées par de 0,01$\mu$g de PPV-PréS:T ou 10 $\mu$g de PPV (VP2), en absence d'adjuvant.

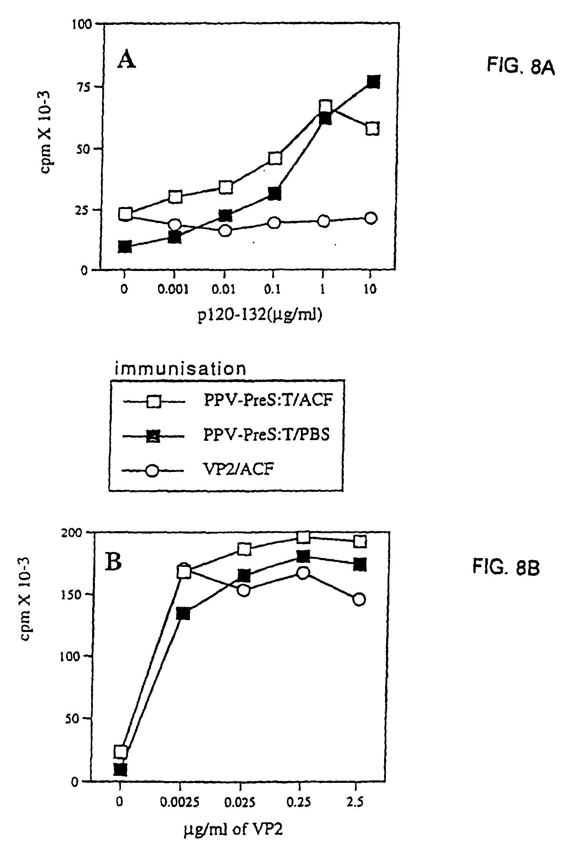

Les figures 8A et 8B représentent la prolifération de cellules ganglionnaires, issues de souris DBA/1 immunisées par des PPV-PréS:T en milieu PBS ou en présence d'adjuvant complet de Freund ou PPV (VP2), restimulées par le peptide PréS:T (figure 8A) ou des PPV (VP2) (Figure 8B).

## EXEMPLE 1: INDUCTION DE LA CYTOTOXICITE T LYMPHOCYTAIRE PAR DES PSEUDO-PARTICULES SELON L'INVENTION PORTANT UN EPITOPE DE LA NUCLEOPROTEINE DE LCMV

L'épitope CTL hétérologue, CD8[+] contenu dans la région 118-132 de la nucléoprotéine de LCMV a été introduit dans le gène de VP2 du PPV sans altérer la formation ultérieure des pseudo-particules. Deux particules ont été obtenues, PPV-29 LCMV et PPV-30 LCMV, différant uniquement par un codon d'initiation. Ces protéines chimères ont été produites par un système de baculovirus et purifiées par précipitation au sulfate d'ammonium à partir du lysat de cellules d'insectes selon la méthode décrite dans Journal of General Virology 76 : pp. 2361-2368 (1995).

La capacité de ces particules recombinantes à induire des réponses lymphocytaires T cytotoxiques a été analysée *in vivo* chez la souris BALB/c.

### Matériel et méthodes

### * Insertion de l'épitope LCMV CD8[+] dans le site Xhol de l'extrémité N-terminale de VP2.

Comme l'illustre la figure 1, le vecteur pPPV29 est obtenu par digestion et remplacement du fragment Xhol - Ncol du vecteur pPPV17R, issu du vecteur pPPV17 décrit par Martinez et al. (1992, Vaccine, 10, 684-690) par deux produits d'amplification PCR, afin d'éliminer une région présente dans le site de polyclonage de pPPV17R et le codon d'initiation de VP2. Les vecteurs pPPV17 et pPPV17R diffèrent uniquement par l'orientation du gène cloné VP2 par rapport au polylinker. Toutes les amplifications PCR ont été effectuées dans un volume total de 100 $\mu$l avec une unité d'ADN polymérase Vent (New England Biolabs), 10 ng de pPPV17R comme matrice, 100 $\mu$m de dNTPs et 800 ng de chaque amorce. Les amplifications ont été effectuées durant 25 cycles de dénaturation à 93°C durant une minute, l'anelage de l'amorce étant effectué à 50°C durant une minute et l'extension à 72°C durant trois minutes. Les produits de la PCR ont été clonés dans le pPPV17R digéré par Xho-I et Ncol et déphosphorylé. Les mélanges de ligature ont été utilisés pour transformer les bactéries E. coli DH5. De cette manière deux plasmides ont été obtenus: pPPV29 qui contient un unique site de clonage Xho-I adjacent au codon ATG originel de VP2, et pPPV30 qui contient un site unique de clonage Xho-I, mais sans le codon d'initiation ATG.

Le vecteur pPPV29 mod a alors été obtenu: le gène VP2 a été obtenu par digestion du pPPV29 par Pst I puis ligaturé dans le site de clonage PstI du plasmide pMTL24 afin de fournir les sites BamH1 pour le sous clonage dans les vecteurs de transfert des baculovirus.

Deux oligonucléotides codant pour l'épitope CD8[+] et le codon d'initiation, et comprenant deux sites Xhol ont été synthétisés. Ils présentent les séquences suivantes: SEQ ID N°1 et N°2 suivantes:

## SEQ ID N°1

5'TCGAGATGCGACCACAAGCTTCAGGAGTATACATGGGAAACCTAACAGCACAAC3'

## SEQ ID N°2

5'TCGAGTTGTGCTGTTAGGTTTCCCATGTATACTCCTGAAGCTTGTGGTCGCATC3

Ces deux oligonucléotides complémentaires ont été fournis par MedProbe (Norvège).Ils ont été phosphorylés à l'aide de la polynucléotide kinase T4, annelés à 70°C durant 15 minutes et ligaturés dans le site XhoI du plasmide pPPV29mod digéré par la XhoI; qui contient le gène codant pour la PPV-VP2.

Des cellules d'*Escherichia coli* DH5 ont été transformées avec le mélange de ligature et étalées sur du milieu LB contenant 100 $\mu$g/ml d'ampicilline (Hanahan, 1983, J. Mol. Biol., 166, 557-580. Les recombinants contenant l'insert LCMV ont été sélectionnés, puis séquencés par le procédé à la didéoxy afin de déterminer l'orientation et l'intégrité des séquences insérées. Le clone recombinant contenant la séquence LCMV dans l'orientation adéquate a été appelé pPPV29mod/LCMV.

## * Construction de vecteurs de transfert dans les baculovirus et sélection des baculovirus recombinants.

La séquence VP2 chimérique a été obtenue à partir du vecteur pPPV29mod/LCMV par digestion par BamHI et sous-clonée dans l'unique site de restriction BamHI du vecteur de transfert du baculovirus PAcYM1.

Des clones recombinants ont été préparés comme décrit ci-dessus et analysés par la méthode de minipréparation d'ADN plasmidique et par cartographie de restriction. Les séquences d'insertion des clones positifs ont été séquencées afin de vérifier à nouveau l'intégrité de l'épitope inséré ainsi que des séquences flanquantes. Finalement, l'ADN purifié par le phénol a été précipité par deux volumes d'éthanol. Le clone recombinant ainsi obtenu a été appelé pAcYM1/LCMV/ppv29mod.

Afin d'obtenir des baculovirus recombinants, un mélange de 2 $\mu$g d'ADN de vecteur de transfert purifié et de 500 ng d'ADN parental AcRP23lacz+ a été ajouté à des cellules d'insectes Sf9 en présence du réactif de transfection DOTAP (Boehringer Mannheim), selon les instructions de cette Société. La transfection a été poursuivie jusqu'à ce que l'effet cytopathique soit total. Après 9 jours, les cultures ont été récupérées et le surnageant clarifié a été utilisé pour l'isolement de plages de baculovirus recombinants selon les méthodes déjà connues de l'homme du métier et décrit par Sedlik et al. (J. Gen. Virol. 1995, 76, 2361-2368). Les clones recombinants ont été sélectionnés en utilisant leur phénotype blanc (plage blanche: recombinant, plage bleue; phénotype sauvage).

Les baculovirus recombinants ont été purifiés jusqu'à ce qu'aucune plage bleue ne puisse être détectée. Des stocks de virus présentant un titre important de baculovirus recombinant AcNPV.PPV-LCMV (>$10^8$) ont été préparés.

Un clone, appelé AcPP29-LMCV a été déposé le 20 Décembre 1995 sous le n°V 95122022 auprès de l'European Collection of Animal Cell Cultures (ECACC).

## * Analyse des protéines recombinantes.

Les cellules Sf9 ont été infectées avec les baculovirus recombinants à raison d'un taux d'infection de 1 pfu par cellule (une unité formant plage par cellule) et les extraits des cellules ont été collectés 72 heures après infection.

Du tampon de dissociation de protéine a été ajouté à chaque extrait cellulaire, et les mélanges ont été chauffés à 100°C durant 5 minutes puis séparés sur SDS-9% PAGE.

Les gels ont été colorés à l'aide de bleu de Coomassie ou transférés sur des membranes de nitrocellulose à l'aide d'un équipement semi-sec, à 22 volts durant 30 minutes.

Les membranes ont été incubées avec du sérum de souris dirigé à l'encontre de l'épitope LCMV CD8[+] (dilution 1/200) durant deux heures à température ambiante.

Après lavage les anticorps fixés ont été détectés par la protéine A conjuguée à la péroxydase (dilution 1/2000) en utilisant du 4-chloronaphtol comme substrat jusqu'à développement de la couleur. Les membranes ont ensuite été rincées avec de l'eau distillée afin d'arrêter la réaction. Après coloration des gels SDS par du bleu de Coomassie, une bande visible a 67 kD a été détectée dans les extraits de cellules Sf9 infectées avec les baculovirus recombinants. La taille observée correspond à la taille attendue pour la protéine chimérique VP2-LCMV. L'identité de cette protéine est confirmée par immunotransfert. L'antisérum de souris spécifique montre une réaction positive très claire avec la protéine de 67 kD des extraits cellulaires infectés. La localisation intracellulaire de la protéine recombinante a été analysée par immunotransfert de différentes fractions cellulaires. Ceci a permis de montrer que la protéine chimérique LCMV-VP2 est une protéine cytoplasmique soluble.

**\* Purification des particules**

Des cellules Sf9 ont été infectées à l'aide de baculovirus recombinant à un taux d'infection de 1 pfu par cellule. Les cellules ont été récoltées 72 heures après l'infection, lavées avec du PBS et lysées par choc osmotique à 4°C dans une solution de bicarbonate 25 mM. Les débris cellulaires ont été éliminés par centrifugation à basse vitesse. Les particules contenues dans les extraits ont été alors purifiées par précipitation par une solution saturée à 20% de sulfate d'ammonium. Le précipitat est centrifugé à 15000 rpm durant 15 minutes, resuspendu dans du PBS et dialysé durant une nuit contre ce même tampon. L'identité et les propriétés des particules ont été confirmées par SDS-PAGE, immunotransfert et par microscopie électronique.
Ces particules purifiées ont été utilisées pour l'immunisation et l'induction de la cytotoxicité lymphocytaire (CTL).

**\* Immunisation des souris.**

Les souris reçoivent par voie intrapéritonéale une injection d'une composition contenant les pseudo-particules virales recombinantes conformes à l'invention (PPV-LCMV), ou vides (PPV), en l'absence d'adjuvant. Les témoins positifs sont des souris injectées, à j21, par voie sous-cutanée avec le peptide synthétique 118-126 (fourni par la société Neosystem, Strasbourg, France) en adjuvant incomplet de Freund (IFA). Des témoins négatifs reçoivent des pseudo-particules n'exprimant pas l'épitope LCMV.

**\*Induction de cellules cytotoxiques**

7 à 14 jours après la dernière immunisation, les rates sont prélevées sur les souris immunisées. 25 x 106 cellules immunes sont mises en culture, en présence de 25 x $10^6$ splénocytes irradiés provenant de souris syngéniques naïves, en flacons contenant du milieu nutritif (RPMI 1640, 10% sérum de veau foetal, glutamine et antibiotique) pendant 5 jours à 37°C, 5% $CO_2$.
Les cellules stimulatrices sont sensibilisées *in vitro* avec le peptide synthétique 118-126 à à 0,05 $\mu$M ou avec le peptide synthétique 118-132 (également fourni par la société Neosystem, Strasbourg, France) à 0,5 $\mu$M.

**\*Test de cytotoxicité**

L'activité cytotoxique des lymphocytes effecteurs a été mesurée après 5 jours. Les cellules cibles P815 sont pré-incubées soit avec du milieu nutritif (témoin négatif), soit avec le peptide antigénique synthétique et marquées simultanément par le $^{51}$Cr pendant 1 h., à 37 °C. Ensuite, les lymphocytes effecteurs sont incubés avec les cellules marquées, à différents rapports effecteur / cible (rapport E / T) en milieu nutritif dans des plaques 96 puits à fond rond, pendant 4 à 5 h., à 37 °C. Les cibles sont incubées dans les mêmes conditions avec du milieu pour mesurer la libération spontanée du $^{51}$Cr et avec de l'acide chlorhydrique 1N pour mesurer la libération maximale de $^{51}$Cr. L'activité des lymphocytes cytotoxiques est déterminée par comptage de la radioactivité présente dans les surnageants de culture, correspondant au relargage du $^{51}$Cr par les cellules cibles lysées.

$$\% \text{ lyse spécifique } = \frac{\text{cpm expérimental - cpm spontané}}{\text{cpm maximal - cpm spontané}}$$

Ce calcul est réalisé avec les cellules cibles incubées dans du milieu nutritif (P815) et avec les cellules cibles incubées avec le peptide synthétique (P815 + p118-132), comme représenté par les courbes des Figures 2 à 4.

**Résultats.**

Comme on peut le constater d'après l'examen des Figures 2 à 5, une seule injection de 10 $\mu$g de pseudo-particules recombinantes présentant l'épitope T, CD8[+] du LCMV a permis d'atteindre pratiquement 100 % de lyse, c'est à dire autant que le témoin positif peptide synthétique p118-126 + FIA, ce qui confirme que les pseudo-particules conformes à l'invention permettent l'induction de réponses CTL *in vivo* à un niveau élevé sans adjonction d'adjuvant.
Cette induction est spécifique de l'épitope inséré car aucune réponse T cytotoxique n'est obtenue lorsque les souris sont immunisées par des doses équivalentes de pseudo-particules n'exprimant pas l'épitope LCMV. Les cellules T cytotoxiques ainsi induites lysent spécifiquement les cellules cibles P815 recouvertes par le peptide correspondant à l'épitope inséré.

La figure 5 montre quant à elle que les souris auxquelles des pseudo-particules ont été injectées sont protégées contre l'infection virale à 28 jours, et que 4/5 souris sont protégées à 70 jours.

## EXEMPLE 2:

**Induction de réponse T, CD4$^{\pm}$, spécifiques d'un épitope du virus de l'hépatite B par des pseudo-particules de parvovirus.**

**1) Matériels et méthodes**

**Insertion de l'épitope PréS:T de l'HBV à l'extrémité N-terminale de la protéine VP2 du PPV.**

Deux oligonucléotides ont été synthétisés, codant pour l'épitope Prés:T de l'HBV, ayant les séquences suivantes:

### Seq ID N°3:
5'TCGAGATGCAATGGAATTCTACCACCTTCCACCAAACCCTGCAAC3'

### SEQ ID N°4:
5'TCGAGTTGCAGGGTTTGGTGGAAGGTGGTAGAATTCCATTGCATC 3'

Ces deux oligonucléotides sont complémentaires et contiennent deux séquences flanquantes du site XhoI aux extrémités afin de permettre un clonage directe dans le vecteur pPPV30. Les oligonucléotides ont été fournis par ISOGEN (Pays-Bas). Ils ont été phosphorylés à l'aide de la polynucléotide kinase T4, annelés à 70°C durant 15 minutes puis ligaturés en présence de pPPV30 digéré par XhoI et traité par de la phosphatase à 14°C durant une nuit. Le mélange a été utilisé pour transformer des cellules *d'Escherichia coli* (souche DH5).

Les colonies ont été analysées à l'aide des enzymes de restriction EcoRI et Hind III afin de vérifier la présence des séquences insérées. Les recombinants contenant la séquence de l'épitope ont été séquencés afin de confirmer qu'aucun changement ou qu'aucune mutation n'est intervenu durant ces manipulations.

**\* Construction d'un vecteur de transfert des constructions PreS:T-PPV dans les baculovirus et obtention de baculovirus recombinants.**

Les plasmides recombinants contenant l'épitope PréS:T dans une orientation correcte ont été digérés à l'aide de BamH1 et la VP2 modifiée a été sous-clonée dans le vecteur de transfert pAcYM1 (Matsuura et al., 1987, J. Gen. Virol. 68, 1233-1250). Les clones recombinants ont été analysés comme décrit précédemment et les séquences des insertions ont été confirmées par séquençage. Le clone recombinant est appelé pAcYM1/PréS:T-pPPV30.

Les baculovirus recombinants ont été obtenus par transfection de cellules d'insectes SF9 , comme décrit dans l'exemple 1 et des stocks de virus présentant des titres importants ont été préparés (> 10$^8$ pfu/ml). Le virus recombinant a été appelé AcPPV30-PréS:T.

Il a été déposé auprès de l'European Collection of Animal Cell Cultures (ECACC).sous le n° V96081412 le 15 Août 1996.

**\* Analyse et purification des protéines recombinantes.**

Des cellules Sf9 ont été infectées (Smith et al., 1983, Mol. Cell. Biol., 3, 2156-2165) avec un taux d'infection de 1pfu/cellule avec le baculovirus recombinant AcPPV30-PréS:T. Des cellules infectées ont été collectées 72 heures après l'infection et analysées par électrophorèse sur des gels de polyacrylamide à 9% en présence de SDS puis par immunotransfert. Les résultats montrent la présence de protéine VP2 recombinante modifiée.

Afin de purifier les particules VP2 chimériques, les cellules infectées ont été lysées à l'aide de bicarbonate de sodium 25mM, les débris cellulaires ont été éliminés par centrifugation et le surnageant contenant les particules a été précipité à l'aide de sulfate d'ammonium à 20% durant 20 minutes à 4°C. Le culot, qui a été enrichi de manière importante en particules VP2, a été collecté par centrifugation et dialysé à nouveau contre du PBS durant une nuit. Les particules purifiées ont été stockées à 4°C jusqu'à utilisation.

**\* Stimulation d'hybridomes T spécifiques du peptide PréS:T de HBV par des pseudo-particules exprimant ce peptide.**

$10^5$ cellules du lymphome B, M12C10(H-$2^{d/q}$) (fig.6A et B) ou $5.10^5$ splénocytes irradiés de souris DBA/1(H-$2^q$)(figures 6C et D) ont été incubés à 37°C , en présence de différentes concentrations de peptide PréS:T correspondant à la séquence 120-132, de pseudo-particules recombinantes PPV-PréS:T, ou de pseudo-particules PPV (VP2), puis ont été utilisées pour stimuler $10^5$ hybridomes T, 51E12 (6A et 6C) ou 52A12 (6B et 6D), spécifiques du peptide PréS:T et restreints par les molécules I-A$^q$; après 24 heures, les surnageants de culture ont été prélevés, puis dosés avec la lignée CTLL dépendante de l'IL-2. Trois jours plus tard, la prolifération des cellules CTLL est mesurée par incorporation de thymidine tritiée.

**\*Induction en absence d'adjuvant de réponses prolifératives spécifiques du peptide PréS:T de HBV après immunisation de souris avec des pseudo-particules virales de parvovirus exprimant ce peptide.**

Des souris DBA/1 (H-$2^q$) ont été immunisées par voie sous-cutanée avec 10μg, 1μg, 0,1μg ou 0,01μg de pseudo-particules virales recombinantes PPV-PréS:T, ou 10μg de pseudo-particules virales PPV (VP2) . Dix jours plus tard, les cellules des ganglions drainants ont été restimulées in vitro avec différentes concentrations de peptide PréS:T (Fig.76A) ou avec 0,5 μg/ml de PPV (VP2) (Fig.7B). Quatre jours plus tard, la prolifération des cellules ganglionnaires est mesurée par incorporation de thymidine tritiée.

**\*Capacité des pseudo-particules virales recombinantes PPV-PréS:T à induire une réponse proliférative spécifique du peptide PréS:T en présence ou en absence d'adjuvant.**

Des souris DBA/1 (H-$2^q$) ont été immunisées par voie sous-cutanée avec 10μg de pseudo-particules virales recombinantes PPV-PréS:T en solution saline (PBS) ou en émulsion avec de l'adjuvant complet de Freund (ACF), ou avec 10μg de pseudo-particules virales PPV (VP2) en ACF. Dix jours plus tard, les cellules des ganglions drainants ont été restimulées in vitro avec différentes concentrations de peptide PréS:T (figure 8A) ou de PPV (VP2) (figure 8B). Quatre jours plus tard, la prolifération des cellules ganglionnaires est mesurée par incorporation de thymidine tritiée.

**2) Résultats**

L'épitope T, CD4$^+$ PréS:T situé dans la région PréS2 (correspondant à la séquence 120-132) du virus HBV a été introduit dans la protéine VP2 du parvovirus. Les pseudo-particules contrôles PPV (VP2) ou recombinantes PPV-PréS:T ont été purifiées.

Dans un premier temps, l'antigénicité de ces particules a été analysée in vitro vis-à-vis d'hybridomes T spécifiques de l'épitope PréS:T. Comme il est visible sur la figure 6, les pseudo-particules virales contenant l'épitope PréS:T stimulent très fortement la production d'IL-2 par ces hybridomes T spécifiques, et ceci de façon spécifique par rapport aux pseudo-particules contrôles. Si l'on compare en molarité l'efficacité des PPV-PréS:T par rapport au peptide p120-132, on constate que les pseudo-particules sont 100 à 1000 fois plus antigéniques que le peptide.

L'immunogénicité de ces particules a ensuite été testée in vivo, soit seules à diverses doses (0,01 à 10μg) (figure 7), soit à 10μg en présence ou non d'adjuvant complet de Freund (figure 8). Ces expériences ont démontré la très forte immunogénicité des pseudo-particules PPV-PréS:T qui, en l'absence de tout adjuvant et à des doses faibles (1 seule injection de 1 μg), induisent des réponses prolifératives très élevées contre l'épitope inséré (figure 7). Ces réponses sont aussi fortes quand les pseudo-particules sont injectées seules que lorsqu'elles sont administrées en présence d'adjuvant de Freund (figure 8).

EXEMPLE 3

Construction de pseudo-particules portant plusieurs épitopes.

Deux oligonucléotides présentant les séquences complémentaires SEQ ID N°5 et SEQ ID N°6 ont été fabriqués:

## SEQ ID N°5 (Mut1 +):
5'TCGAGATGTTCGAACAAAACACCGCTCAACCCC 3'

## SEQ ID N°6 (Mut 1) :
5'TCGAGGGGTTGAGCGGTGTTTTGTTCGAACATC 3'.

Les extrémités 5' de chacun de ces oligonucléotides peuvent se lier ensemble afin de générer plusieurs copies de l'épitope. Les oligonucléotides ont été phosphorilés, puis mélangés et chauffés à 70°C durant 15 minutes avant ligature dans le plasmide pPPV29mod traité par de la phosphatase alcaline intestinale de veau, et digéré par Xho1.

Les mélanges de ligature ont été utilisés pour transformer des bactéries *E. coli* DH5α. Les clones recombinants ont été caractérisés par analyse à l'aide d'enzymes de restriction. L'incorporation d'une ou plusieurs copies, ou l'absence d'incorporation de l'épitope Mut1 a été mise en évidence par la détermination de la taille du fragment BamH1/HindIII. L'intégrité des séquences de l'épitope et leurs orientations ont été confirmées par séquençage. Les gènes VP2 modifiés contenant les insertions des épitopes Mut1 ont été digérés par BamH1 et sous-clonés dans le vecteur de transfert des baculovirus pAcYM1.

Des baculovirus recombinants ont été obtenus à partir de cotransfections de 500 ng d'ADN viral AcRP23-lacZ linéarisés par Bsu36I et de 2 μg de vecteurs de transfert individuels en utilisant la technique de la lipofectine décrite par Felgner et al. (1987, Proceedings of the National Academy Sciences, USA 84, 7413-7417).

Les virus recombinants ont été sélectionnés sur la base de leurs phénotypes lacz-négatifs et purifiés par plaque avant la préparation de stocks viraux présentant un titre important pour chacun des virus recombinants.

**Revendications**

1.  Pseudo-particules virales recombinantes **caractérisées en ce qu'**elles sont constituées par auto-assemblage d'au moins la protéine de structure virale VP2 d'un virus de la famille des *Parvoviridae*, ou d'un virus apparenté, **en ce qu'**elles ont une taille comprise entre environ 20 et 60 nm, **en ce qu'**elles forment une structure approximativement icosaédrique, et **en ce que** la protéine VP2 est modifiée, en son extrémité N-terminale, par la présence d'au moins un épitope comprenant une séquence de 8 à 25 acides aminés susceptible de s'associer à au moins une molécule de classe I du complexe majeur d'histocompatibilité, ladite association étant reconnue par des lymphocytes T cytotoxiques.

2.  Pseudo-particules selon la revendication 1 **caractérisées en ce que** ledit épitope comprend une séquence de 8 ou 9 acides aminés susceptible de s'associer à au moins une molécule de classe I.

3.  Pseudo-particules selon l'une des revendications 1 et 2, **caractérisées en ce que** ledit parvovirus est le PPV, le CPV ou autre virus apparenté tel que le FPLV et le MEV.

4. Pseudo-particules selon l'une des revendications 1 à 3, **caractérisées en ce que** ledit épitope est un épitope de la nucléoprotéine du virus de la chorioméningite lymphocytaire.

5. Pseudo-particules selon l'une des revendications 1 à 4, **caractérisées en ce que** ledit épitope est l'épitope CTL CD8$^+$ contenu dans la région 118-132 de la nucléoprotéine du virus de la chorioméningite lymphocytaire.

6. Pseudo-particules selon l'une des revendications 1 à 5, **caractérisées en ce qu'**elles comprennent au moins un épitope TCD4$^+$ et au moins un épitope TCD8$^+$.

7. Pseudo-particules selon les revendications 1 à 6, **caractérisées en ce qu'**elles comprennent une combinaison de multicopies d'épitopes TCD8$^+$.

8. Pseudo-particules selon les revendications 1 et 6, **caractérisées en ce qu'**elles comprennent une combinaison de quatre copies d'épitopes TCD8$^+$ Mut 1 du carcinome de LEWIS.

9. Pseudo-particules selon l'une quelconque des revendications 1 à 8, **caractérisées en ce qu'**elles sont susceptibles d'être obtenues par un procédé comprenant une étape d'expression dans le baculovirus de la protéine hybride formée de la protéine VP2 de parvovirus et dudit épitope.

10. Composition comprenant des pseudo-particules selon l'une quelconque des revendications 1 à 9, dans un véhicule et/ou un diluant physiologiquement acceptable.

11. Composition selon la revendication 10, **caractérisée en ce qu'**elle ne comprend pas d'adjuvant immunostimulateur.

12. Utilisation des pseudo-particules selon l'une quelconque des revendications 1 à 9, ou d'une composition selon l'une des revendications 10 et 11 pour la fabrication de médicaments ou de vaccins pour l'induction de réponses CTL ou de cytokines *in vivo*.

13. Utilisation des pseudo-particules selon l'une quelconque des revendications 1 à 9, ou d'une composition selon l'une des revendications 10 et 11 dans la fabrication de vaccins, en particulier de vaccins antiviraux ou de médicaments antitumoraux.

14. Procédé de stimulation *in vitro* des réponses T cytotoxiques de lymphocytes provenant de sujets atteints d'infections virales ou tumorales comprenant la mise en contact des lymphocytes des sujets avec les pseudo-particules selon l'une des revendications 1 à 9.


**Patentansprüche**

1. Rekombinante Pseudoviruspartikel, **dadurch gekennzeichnet, dass** sie durch Selbstorganisation wenigstens des viralen Strukturproteins VP2 eines Virus der Familie der Parvoviridae oder eines verwandten Virus entstehen, dass sie eine Größe zwischen etwa 20 und 60 nm haben, dass sie eine ungefähr icosaedrische Struktur bilden und dass das Protein VP2 an seinem N-Terminus durch die Anwesenheit wenigstens eines Epitops modifiziert ist, das eine Sequenz von 8 bis 25 Aminosäuren umfasst, die sich an wenigstens ein Molekül der Klasse I des Haupthistokompatibilitätskomplexes assoziieren kann, wobei die Assoziation von cytotoxischen T-Lymphocyten erkannt wird.

2. Pseudopartikel gemäß Anspruch 1, **dadurch gekennzeichnet, dass** das Epitop eine Sequenz von 8 oder 9 Aminosäuren umfasst, die sich an wenigstens ein Molekül der Klasse I assoziieren kann.

3. Pseudopartikel gemäß einem der Ansprüche 1 und 2, **dadurch gekennzeichnet, dass** es sich bei dem Parvovirus um PPV, CPV oder ein anderes, verwandtes Virus, wie FPLV und MEV, handelt.

4. Pseudopartikel gemäß einem der Ansprüche 1 bis 3, **dadurch gekennzeichnet, dass** das Epitop ein Epitop des Kernproteins des lymphozytären Choriomeningitis-Virus ist.

5. Pseudopartikel gemäß einem der Ansprüche 1 bis 4, **dadurch gekennzeichnet, dass** das Epitop das CD8-positive CTL-Epitop ist, das im Bereich 118-132 des Kernproteins des lymphozytären Choriomeningitis-Virus enthalten ist.

**6.** Pseudopartikel gemäß einem der Ansprüche 1 bis 5, **dadurch gekennzeichnet, dass** sie wenigstens ein CD4-positives T-Zell-Epitop und wenigstens ein CD8-positives T-Zell-Epitop umfassen.

**7.** Pseudopartikel gemäß den Ansprüchen 1 bis 6, **dadurch gekennzeichnet, dass** sie eine Kombination von mehrfachen Kopien von CD8-positiven T-Zell-Epitopen umfassen.

**8.** Pseudopartikel gemäß den Ansprüchen 1 bis 6, **dadurch gekennzeichnet, dass** sie eine Kombination von vier Kopien der CD8-positiven T-Zell-Epitope Mut 1 des Lewis-Karzinoms umfassen.

**9.** Pseudopartikel gemäß einem der Ansprüche 1 bis 8, **dadurch gekennzeichnet, dass** sie durch ein Verfahren erhalten werden können, das einen Schritt der Expression des aus dem Protein VP2 von Parvovirus und dem Epitop gebildeten Hybridproteins in Baculovirus umfasst.

**10.** Zusammensetzung, die Pseudopartikel gemäß einem der Ansprüche 1 bis 9 in einem physiologisch annehmbaren Träger und/oder Verdünnungsmittel umfasst.

**11.** Zusammensetzung gemäß Anspruch 10, **dadurch gekennzeichnet, dass** sie kein immunstimulierendes Adjuvans umfasst.

**12.** Verwendung der Pseudopartikel gemäß einem der Ansprüche 1 bis 9 oder einer Zusammensetzung gemäß einem der Ansprüche 10 und 11 zur Herstellung von Medikamenten oder Impfstoffen zur Induktion von CTL-Antworten oder von Cytokinen in vivo.

**13.** Verwendung der Pseudopartikel gemäß einem der Ansprüche 1 bis 9 oder einer Zusammensetzung gemäß einem der Ansprüche 10 und 11 bei der Herstellung von Impfstoffen, insbesondere antiviralen Impfstoffen, oder von Antitumormedikamenten.

**14.** Verfahren zur in-vitro-Stimulation der cytotoxischen T-Antworten von Lymphocyten, die von Patienten stammen, die eine virale oder tumorale Infektion erlitten haben, umfassend das In-Kontakt-Bringen der Lymphocyten der Patienten mit den Pseudopartikeln gemäß einem der Ansprüche 1 bis 9.

**Claims**

**1.** Recombinant viral pseudoparticles, **characterized in that** they are formed by self-assembly of at least the viral structural protein VP2 of a virus of the family *Parvoviridae*, or of a related virus, **in that** they have a size of between about 20 and 60 nm, **in that** they form an approximately icosahedral structure, and **in that** the VP2 protein is modified, at its N-terminal end, by the presence of at least one epitope comprising a sequence of 8 to 25 amino acids liable to be combined with at least one major histocompatibility complex class I molecule, said association being recognized by cytotoxic T lymphocytes.

**2.** Pseudoparticles according to Claim 1, **characterized in that** said epitope comprises a sequence of 8 or 9 amino acids liable to be combined with at least one class I molecule.

**3.** Pseudoparticles according to either of Claims 1 and 2, **characterized in that** said parvovirus is PPV, CPV or another related virus such as FPLV and MEV.

**4.** Pseudoparticles according to one of Claims 1 to 3, **characterized in that** said epitope is an epitope of the lymphocytic choriomeningitis virus nucleoprotein.

**5.** Pseudoparticles according to one of Claims 1 to 4, **characterized in that** said epitope is the CD8+ CTL epitope contained in region 118-132 of the lymphocytic choriomeningitis virus nucleoprotein.

**6.** Pseudoparticles according to one of Claims 1 to 5, **characterized in that** they comprise at least one CD4+ T epitope and at least one CD8+ T epitope.

**7.** Pseudoparticles according to Claims 1 to 6, **characterized in that** they comprise a combination of multicopies of CD8+ T epitopes.

8. Pseudoparticles according to Claims 1 to 6, **characterized in that** they comprise a combination of 4 copies of LEWIS carcinoma Mut 1 CD8+ T epitopes.

9. Pseudoparticles according to any one of Claims 1 to 8, **characterized in that** they can be obtained by means of a method comprising a step consisting of expression, in the baculovirus, of the hybrid protein made up of the parvovirus VP2 protein and of said epitope.

10. Composition comprising pseudoparticles according to any one of Claims 1 to 9, in a physiologically acceptable vehicle and/or diluent.

11. Composition according to Claim 10, **characterized in that** it comprises no immunostimulatory adjuvant.

12. Use of the pseudoparticles according to any one of Claims 1 to 9, or of a composition according to either of Claims 10 and 11, for the production of medicinal products or of vaccines for inducing CTL responses or cytokines *in vivo*.

13. Use of the pseudoparticles according to any one of Claims 1 to 9, or of a composition according to either of Claims 10 and 11, in the production of vaccines, in particular antiviral vaccines or of antitumour medicinal products.

14. Method of stimulating, in vitro, the cytotoxic T responses of lymphocytes originating from individuals suffering viral or tumoral infections, which comprising bringing the lymphocytes of the individuals into contact with the pseudoparticles according to one of Claims 1 to 9.

# FIG. 1

FIG. 2A — PPV-29 LCMV

FIG. 2B — PPV-30 LCMV

FIG. 2C — PPV

RAPPORT E:T

% DE LYSE

10 µg PPV — P815

10 µg PPV — P815 + p118-132

50 µg PPV — P815

50 µg PPV — P815 + p118-132

## FIG. 3A

### 50 μg PPV-30 LCMV

## FIG. 3B

### 10 μg PPV-30 LCMV

## FIG. 3C

### 2 μg PPV-30 LCMV

## FIG. 3D

### 0.4 μg PPV-30 LCMV

## FIG. 3E

0.08 μg PPV-30 LCMV

Legend:
- ○ — P815
- ● — P815 + p118-132

## FIG. 3F

10 μg PPV

## FIG. 3G

100 μg118-126+FIA

FIG. 4A
PPV-29LCMV

FIG. 4B
PPV-30LCMV

FIG. 4C
PPV

% DE LYSE

RAPPORT E:T

RAPPORT E:T

RAPPORT E:T

10 μg PPV ——○—— P815          50 μg PPV ——□—— P815

——●—— P815 + p118-132          ——■—— P815 + p118-132

EP 0 871 738 B1

FIG. 5B

FIG. 5A

PBS
10 μg PPV
10 μg PPV-LCMV
LCMV Arms. $10^5$PFU

FIG. 6A

FIG. 6B

FIG. 6C

FIG. 6D

FIG. 7A

FIG. 7B

FIG. 8A

FIG. 8B


LISTE DE SEQUENCES

(1) INFORMATIONS GENERALES:

(i) DEPOSANT:

(A) NOM: INSTITUT PASTEUR
(B) RUE: 25-28, rue du Docteur Roux
(C) VILLE: PARIS
(E) PAYS: FRANCE
(F) CODE POSTAL: 75724 PARIS
(G) TELEPHONE: 45 68 80 93
(I) TELEX: 250 609

(A) NOM: IMMUNOLOGIA Y GENETICA APLICADA, S.A.
(B) RUE: Hermanos Garcia Noblejas, 41 20
(C) VILLE: MADRID
(E) PAYS: ESPAGNE
(F) CODE POSTAL: E-28037

(ii) TITRE DE L' INVENTION: Pseudo-particules virales recombinantes et applications vaccinales et antitumorales

(iii) NOMBRE DE SEQUENCES: 6

(iv) FORME DECHIFFRABLE PAR ORDINATEUR:

(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(2) INFORMATIONS POUR LA SEQ ID NO: 1:

    (i) CARACTERISTIQUES DE LA SEQUENCE:

        (A) LONGUEUR: 54 paires de bases
        (B) TYPE: nucléotide
        (C) NOMBRE DE BRINS: simple
        (D) CONFIGURATION: linéaire

    (ii) TYPE DE MOLECULE: ADN (génomique)
    (xi) DESCRIPTION DE LA SEQUENCE: SEQ ID NO: 1:

        TCGAGATGCG ACCACAAGCT TCAGGAGTAT ACATGGGAAA CCTAACAGCA CAAC   54

(2) INFORMATIONS POUR LA SEQ ID NO: 2:

    (i) CARACTERISTIQUES DE LA SEQUENCE:

        (A) LONGUEUR: 54 paires de bases
        (B) TYPE: nucléotide
        (C) NOMBRE DE BRINS: simple
        (D) CONFIGURATION: linéaire

    (ii) TYPE DE MOLECULE: ADN (génomique)
    (xi) DESCRIPTION DE LA SEQUENCE: SEQ ID NO: 2:

        TCGAGTTGTG CTGTTAGGTT TCCCATGTAT ACTCCTGAAG CTTGTGGTCG CATC   54

(2) INFORMATIONS POUR LA SEQ ID NO: 3:

    (i) CARACTERISTIQUES DE LA SEQUENCE:

        (A) LONGUEUR: 45 paires de bases
        (B) TYPE: nucléotide
        (C) NOMBRE DE BRINS: simple
        (D) CONFIGURATION: linéaire

    (ii) TYPE DE MOLECULE: ADN (génomique)
    (xi) DESCRIPTION DE LA SEQUENCE: SEQ ID NO: 3:

        TCGAGATGCA ATGGAATTCT ACCACCTTCC ACCAAACCCT GCAAC   45

(2) INFORMATIONS POUR LA SEQ ID NO: 4:

    (i) CARACTERISTIQUES DE LA SEQUENCE:

        (A) LONGUEUR: 45 paires de bases
        (B) TYPE: nucléotide
        (C) NOMBRE DE BRINS: simple
        (D) CONFIGURATION: linéaire

(ii) TYPE DE MOLECULE: ADN (génomique)
(xi) DESCRIPTION DE LA SEQUENCE: SEQ ID NO: 4:


TCGAGTTGCA GGGTTTGGTG GAAGGTGGTA GAATTCCATT GCATC          45


(2) INFORMATIONS POUR LA SEQ ID NO: 5:

(i) CARACTERISTIQUES DE LA SEQUENCE:

(A) LONGUEUR: 33 paires de bases
(B) TYPE: nucléotide
(C) NOMBRE DE BRINS: simple
(D) CONFIGURATION: linéaire

(ii) TYPE DE MOLECULE: ADN (génomique)
(iii) HYPOTHETIQUE: NON
(xi) DESCRIPTION DE LA SEQUENCE: SEQ ID NO: 5:


TCGAGATGTT CGAACAAAAC ACCGCTCAAC CCC          33


(2) INFORMATIONS POUR LA SEQ ID NO: 6:

(i) CARACTERISTIQUES DE LA SEQUENCE:

(A) LONGUEUR: 33 paires de bases
(B) TYPE: nucléotide
(C) NOMBRE DE BRINS: simple
(D) CONFIGURATION: linéaire

(ii) TYPE DE MOLECULE: ADN (génomique)
(iii) HYPOTHETIQUE: NON
(xi) DESCRIPTION DE LA SEQUENCE: SEQ ID NO: 6:


TCGAGGGGTT GAGCGGTGTT TTGTTCGAAC ATC          33